# EUROPEAN PATENT APPLICATION

(11) **EP 4 410 208 A1**
(43) Date of publication of application: **07.08.2024**
(21) Application number: 22876057.5
(22) Date of filing: 22.09.2022
(51) Int. Cl.: A61B 5/259, A61B 5/274

(54) **BIOELECTRODE**

(30) Priority: 28.09.2021 JP 2021157875
(71) Applicant: Sekisui Kasei Co., Ltd., Kita-ku Osaka-shi Osaka 530-8565 (JP)
(72) Inventor: FUJITA, Takahiko, Osaka-shi, Osaka 530-8565 (JP); HATORI, Takaaki, Osaka-shi, Osaka 530-8565 (JP)
(74) Representative: Müller-Boré & Partner Patentanwälte PartG mbB
(86) International application number: PCT/JP2022/035469
(87) International publication number: WO 2023/054190

(57) **Abstract**

Provided is a bioelectrode including: a substrate plate having a plurality of through holes spaced apart from each other; a plurality of electrode parts; and a fixing plate mounted to a protruding portion of a connection terminal. Each electrode part includes: a covering layer; a connection terminal which is conductive and passes through the covering layer; and a conductive gel layer which is electrically connected to the connection terminal, and is to be brought into contact with a living body. The connection terminal includes a protruding portion protruding from the covering layer, and the protruding portion passes through one of the plurality of through holes of the substrate plate. A size of each of the plurality of through holes of the substrate plate is a size that allows the protruding portion of the connection terminal to be inserted therethrough, and is smaller than a size of the fixing plate.

## Description

### Technical Field

This invention relates to a bioelectrode, and more specifically, to a bioelectrode to be put on a living body in order to acquire a signal from the living body or to perform various types of therapy or treatment on the living body.

### Background Art

In order to transmit an electrical signal from a living body to a measurement device or transmit electrical stimulation from a stimulation device to the living body, there has been used a bioelectrode including a conductive layer to be attached to a skin surface of the living body.

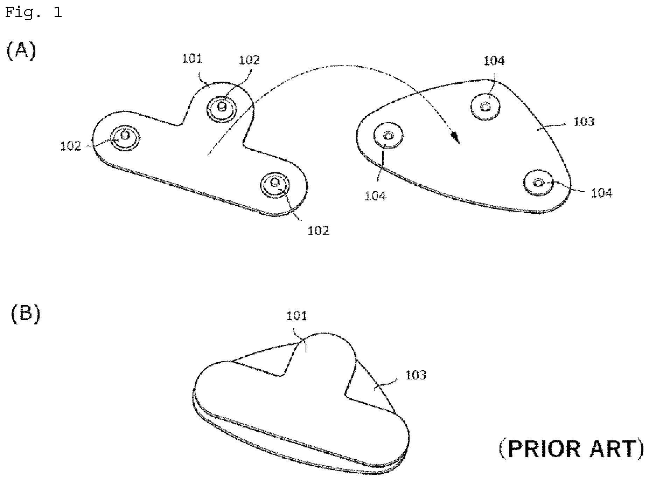

As such a bioelectrode, for example, a pad 101 as illustrated in FIG. 1(A) has been used from a long time ago. The pad 101 includes a plurality of electrodes provided on a support, and metal male hooks 102 that are electrically continuous with the plurality of electrodes. The pad 101 is of a type in which the male hooks 102 of the pad 101 are connected to female hooks 104 of a corresponding connector 103 of an external device. The male hooks 102 and the female hooks 104 are fitted to each other to establish electrical connection as connection terminals. FIG. 1(B) shows a state in which the pad 101 of FIG. 1(A) is moved in the arrow direction so that the male hooks 102 and the female hooks 104 are fitted to each other and thus the pad 101 is mounted to the connector 103. As described above, the bioelectrode including a plurality of electrode parts in one main body is more preferred than a configuration of a bioelectrode having only one electrode in one main body, in order to achieve easiness of handling on a user side and avoid occurrence of unintended troubles or measurement failures.

A connection terminal (male hook) electrically connected to each of the plurality of electrodes of the bioelectrode and a connection terminal (female hook) of the corresponding connector of the external device such as the measurement device or the stimulation device are required to be exactly fitted to each other, and hence position accuracy of the connection terminals is required. Incidentally, in some cases, a distance between the connection terminals may have a slight deviation due to an error at the time of manufacture of the bioelectrode and the measurement device or the stimulation device, or due to long-term usage. Further, a distance between connection terminals of the bioelectrode may vary depending on a site of the living body to which the bioelectrode is applied. Thus, it is preferred that the distance between the connection terminals of the bioelectrode be changeable.

In Patent Literature 1, there is disclosed a pad for a bioelectrode in which a fragile portion is formed between electrode parts of a holder so that the distance between the electrode parts can be changed by cutting the fragile portion. In this pad for a bioelectrode, an interval between the two electrode parts is ordinarily kept constant, but the distance between the electrode parts can be changed by cutting the fragile portion as required.

In Patent Literature 2, there is disclosed a sheet for a bioelectrode including a sheet-shaped support and two electrodes provided on one surface of the support. The sheet for a bioelectrode has, between the two electrodes, a plurality of grooves extending parallel to each other in a direction intersecting with a direction in which the electrodes are opposed to each other. In this sheet 1 for a bioelectrode, a base substrate serving as a support is mountain-folded and valley-folded through use of the plurality of grooves formed between the two electrodes as a folding portion. Thus, even when a wearer moves, the electrode worn on the living body surface is less liable to come off, and the interval between the two electrodes can be freely adjusted.

### Citation List

### Patent Literature

PTL 1: JP 6181356 B2
PTL 2: JP 2019-072171 A

### Summary of Invention

### Technical Problem

In the pad for a bioelectrode of Patent Literature 1, when the fragile portion formed in the holder is once cut, the pad cannot be restored to its original configuration. In the sheet for a bioelectrode of Patent Literature 2, the pad for a bioelectrode can be expanded or contracted in the direction in which the electrodes are opposed to each other through use of the plurality of grooves as the folding portion, but the expansion/contraction is only in one direction, and the entire pad for a bioelectrode is required to be replaced even when a part of the electrodes is consumed.

This invention has an object to provide a bioelectrode including a plurality of electrodes and a distance between connection terminals of adjacent electrode parts can be changed.

### Solution to Problem

In order to solve the above-mentioned problem, the invention of the present application encompasses the following embodiments.
Item 1. A bioelectrode, including: a substrate plate having a plurality of through holes spaced apart from each other; a plurality of electrode parts, each of the plurality of electrode parts including: a covering layer; a connection terminal which is conductive and passes through the covering layer, the connection terminal including a protruding portion protruding from the covering layer, the protruding portion passing through one of the plurality of through holes of the substrate plate; and a conductive layer which is electrically connected to the connection terminal, and is to be brought into contact with a living body; and a fixing plate mounted to the protruding portion of the connection terminal, wherein a size of each of the plurality of through holes of the substrate plate is a size that allows the protruding portion of the connection terminal to be inserted therethrough, and is smaller than a size of the fixing plate.
Item 2. The bioelectrode according to Item 1, wherein the each of the plurality of electrode parts is movable within a range of a corresponding one of the plurality of through holes of the substrate plate so that a distance between connection terminals of adjacent electrode parts is changeable.
Item 3. The bioelectrode according to Item 1 or 2, wherein the connection terminal and the conductive layer of the each of the plurality of electrode parts are separated from each other, and the each of the plurality of electrode parts further includes a second conductive layer extending between the covering layer and the conductive layer under a state in which the second conductive layer is in contact with the connection terminal and the conductive layer.
Item 4. The bioelectrode according to Item 3, wherein the each of the plurality of electrode parts further includes an insulating layer which is attached to the second conductive layer, and which covers a conductor fitted to the connection terminal or an end portion of the connection terminal on a side opposite to the protruding portion in a direction perpendicular to a direction in which the covering layer extends.
Item 5. The bioelectrode according to any one of Items 1 to 4, wherein the connection terminal includes a male hook.
Item 6. The bioelectrode according to any one of Items 1 to 5, wherein, when the protruding portion of the connection terminal is inserted through a corresponding one of the plurality of through holes of the substrate plate, and the fixing plate is fitted to the protruding portion, under a state in which the plurality of electrode parts are mounted to the substrate plate so as to be unremovable from the substrate plate, in plan view, 50% or more of an area of the conductive layer is arrangeable at a position not overlapping the substrate plate.
Item 7. The bioelectrode according to any one of Items 1 to 6, wherein the conductive layer includes a conductive gel layer.
Item 8. A set for assembling a bioelectrode, including: a substrate plate having a plurality of through holes spaced apart from each other; a plurality of electrode parts mountable to and removable from the substrate plate, each of the plurality of electrode parts including: a covering layer; a connection terminal which is conductive and passes through the covering layer, the connection terminal including a protruding portion protruding from the covering layer; and a conductive layer which is electrically connected to the connection terminal, and is to be brought into contact with a living body; and a fixing plate mountable to the protruding portion of the connection terminal, wherein a size of each of the plurality of through holes of the substrate plate is a size that allows the protruding portion of the connection terminal to be inserted therethrough, and is smaller than a size of the fixing plate.

### Advantageous Effects of Invention

According to the bioelectrode of this invention, the distance between the connection terminals of adjacent electrode parts can be changed.

### Brief Description of Drawings

FIG. 1(A) is a perspective view for illustrating a related-art pad and a corresponding connector of an external device, and FIG. 1(B) is a schematic perspective view for illustrating a state in which the pad of FIG. 1(A) is mounted to the connector of the external device.
FIG. 2 is a schematic exploded perspective view of fixing plates, a substrate plate, and a plurality of electrode parts which form a bioelectrode according to an embodiment of this invention.
FIG. 3 is a schematic perspective view of the bioelectrode in which the fixing plates, the substrate plate, and the plurality of electrode parts are integrated with each other.
FIG. 4 is a schematic perspective view of the bioelectrode of FIG. 3 as viewed from the back side.
FIG. 5(A) is a plan view of the electrode part, FIG. 5(B) is a sectional view of the electrode part taken along the line a-a of FIG. 5(A), and FIG. 5(C) is a back view of the electrode part of FIG. 5(A).
FIG. 6 is a partial side view for illustrating a state in which the fixing plate, the substrate plate, and the electrode part are integrated with each other.
FIG. 7 is a schematic sectional view of the electrode part in another embodiment.
FIG. 8 is a schematic sectional view of the electrode part in another embodiment.
FIG. 9 is a schematic plan view of a bioelectrode according to another embodiment, for illustrating a state in which the fixing plates, the substrate plate, and a plurality of electrode parts of FIG. 7 are integrated with each other.
FIG. 10 is a schematic perspective view of the fixing plate in another embodiment.
FIG. 11 is a schematic perspective view of the fixing plate in another embodiment.
FIG. 12(A) is a plan view of another electrode part, FIG. 12(B) is a sectional view of the electrode part taken along the line a-a of FIG. 12(A), and FIG. 12(C) is a back view of the electrode part of FIG. 12(A).
FIG. 13 is a schematic back-side perspective view of a bioelectrode including a pressure-bonding adhesive layer on a surface of the substrate plate.
FIG. 14 is a schematic plan view of a substrate plate in another example.

### Description of Embodiments

Bioelectrodes according to embodiments of the invention are described with reference to the drawings.

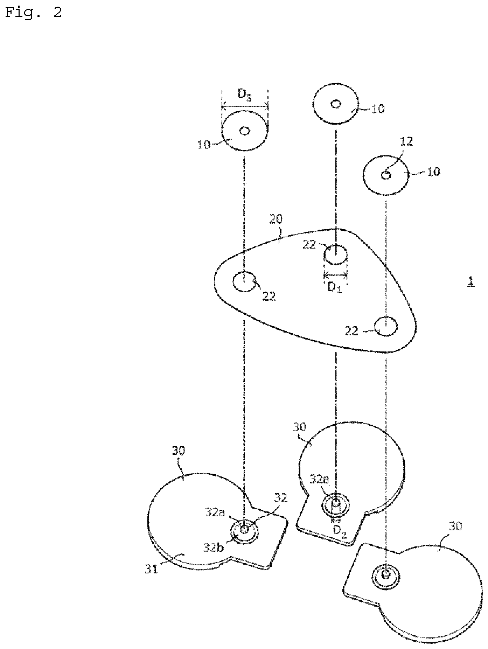

As illustrated in FIG. 2, a bioelectrode 1 according to one embodiment of this invention includes fixing plates 10, a substrate plate 20 having a plurality of (three in the figure) through holes 22 spaced apart from each other, and a plurality of (three in the figure) electrode parts 30. The bioelectrode 1 is formed by assembling these components.

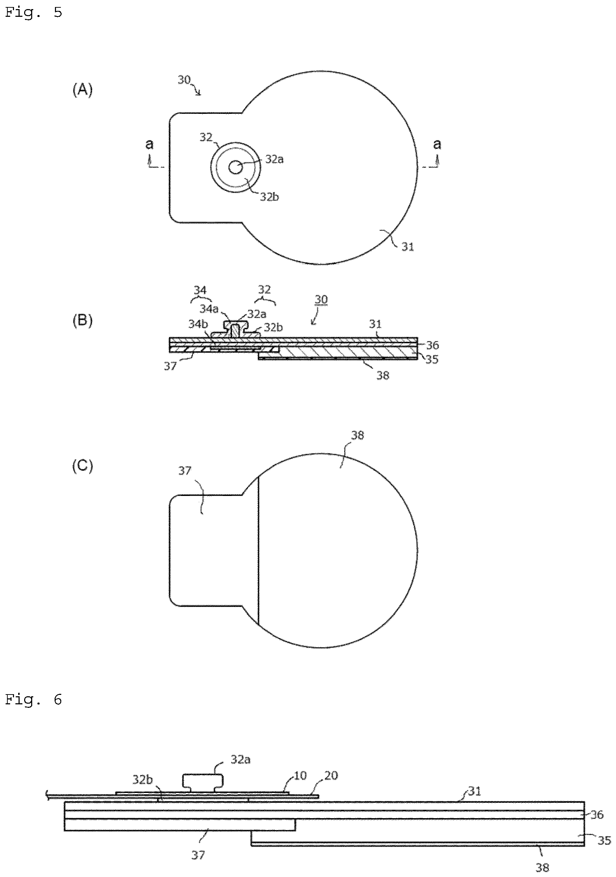

Each electrode part 30 includes a covering layer 31, a connection terminal 32 which is conductive and passes through the covering layer 31, and a conductive gel layer 35 (see FIG. 5(B)) serving as a conductive layer electrically connected to the connection terminal 32. In this embodiment, the connection terminal 32 is a stud of a hook, and a protruding portion 32a of the connection terminal 32 protrudes from the covering layer 31. That is, the protruding portion 32a protrudes to the outside with respect to the surface of the covering layer 31. The fixing plate 10 includes a through hole 12, and can be mounted to the protruding portion 32a of the connection terminal 32 through the through hole 12. The size of each of the plurality of through holes 22 of the substrate plate 20 is a size that allows the protruding portion 32a of the connection terminal 32 to be inserted through the each of the plurality of through holes 22, and is smaller than the size of the fixing plate 10. More specifically, a diameter D₁ of each of the plurality of through holes 22 of the substrate plate 20 is larger than a diameter D₂ of the protruding portion 32a of the connection terminal 32, and is smaller than a diameter D₃ of the fixing plate 10.

The size of the through hole refers to a diameter or a surface area of the through hole when the surface shape of the through hole is a circle. The size of the through hole is determined by the surface area of the through hole when the surface shape of the through hole is not a circle. The size of the fixing plate 10 refers to a surface area of a wide surface of the fixing plate 10.

When the bioelectrode 1 is assembled, first, the protruding portion 32a of the connection terminal 32 of the electrode part 30 is inserted through the corresponding through hole 22 of the substrate plate 20. In this manner, the electrode part 30 is removably mounted to the substrate plate 20. Next, the protruding portion 32a of the electrode part 30 is inserted through the through hole 12 of the fixing plate 10.

In this embodiment, the fixing plate 10 is thin, and the through hole 12 is plastically deformable to some extent. In addition, the protruding portion 32a of the connection terminal 32 of the electrode part 30 has a diameter slightly narrower on a proximal end side than a distal end of the protruding portion 32a. For example, the diameter of the through hole 12 of the fixing plate 10 is the same or smaller than the maximum diameter of the protruding portion 32a. It is preferred that the diameter of the through hole 12 be smaller by 0.1 mm to 1 mm than the maximum diameter of the protruding portion 32a. Accordingly, even after the protruding portion 32a passes through the through hole 12 of the fixing plate 10, unless an external force is applied, the fixing plate 10 stops in the vicinity of a portion at which the diameter is slightly narrower on the proximal end side than the distal end of the protruding portion 32a, and is thus prevented from slipping out from the protruding portion 32a. The fixing plate 10 is fixedly mounted to the protruding portion 32a, and hence the substrate plate 20 is also held between the fixing plate 10 and the electrode part 30 without coming off from the protruding portion 32a, and thus the fixing plate 10, the substrate plate 20, and the one or the plurality of electrode parts 30 are integrated or combined with each other.

When the assembly of the bioelectrode 1 is disassembled, an external pressure is manually applied to the fixing plate 10 and/or the electrode part 30 in a direction of pulling out the fixing plate 10 from the protruding portion 32a of the connection terminal 32 of the electrode part 30. With this, the protruding portion 32a of the connection terminal 32 of the electrode part 30 passes through the through hole 12 of the fixing plate 10 so as to be pulled apart from the fixing plate 10. Then, also the substrate plate 20 can be easily removed from the protruding portion 32a of the connection terminal 32 of the electrode part 30.

In this embodiment, the outer shape of the fixing plate 10 is a substantially circular shape, but the outer shape of the fixing plate 10 is not limited. The outer shape of the fixing plate 10 may be, for example, a circular shape, an elliptical shape, a rectangular shape, or other shapes. It is preferred that the fixing plate 10 be transparent in view of enhancing visibility of the positions of the substrate plate 20 and the electrode part 30, but the fixing plate 10 may be opaque. The material for forming the fixing plate 10 is not limited, but is preferred to be a synthetic resin. Examples of the material include polyethylene terephthalate (PET), polyethylene (PE), and polypropylene (PP). The thickness of the fixing plate 10 should not be too thick because the fixing plate 10 is required to allow the protruding portion 32a of the connection terminal 32 to pass therethrough and is also required to be mounted on the protruding portion 32a. The thickness of the fixing plate 10 is, for example, from 50 um to 200 um, preferably from 75 um to 185 µm.

In this embodiment, the outer shape of the substrate plate 20 is a substantially triangular shape whose sides are rounded, but the outer shape is not particularly limited. The outer shape may be, for example, a circular shape, an elliptical shape, a rectangular shape, or other shapes. Moreover, a tab or a projection for facilitating removal of the substrate plate 20 from the connector of the external device may be provided to the outer shape of any of those shapes. Further, the number of the through holes 22 formed in the substrate plate 20 is not limited to three. It is preferred that the substrate plate 20 be transparent in view of enhancing visibility of the positions of the fixing plate 10 and the electrode part 30, but the substrate plate 20 may be opaque. The material for forming the substrate plate 20 is not limited, but is preferred to be a synthetic resin. Examples of the material include polyethylene terephthalate (PET), polyethylene (PE), and polypropylene (PP). The thickness of the substrate plate 20 is not particularly limited. The thickness of the substrate plate 20 is, for example, from 10 um to 1 mm, preferably from 50 um to 200 µm.

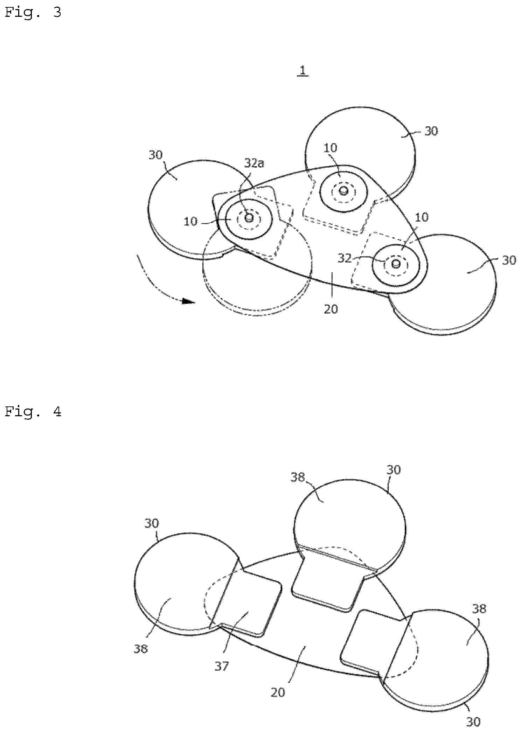

FIG. 3 shows the bioelectrode 1 obtained by integrating or combining the fixing plates 10, the substrate plate 20, and the three electrode parts 30 with each other. Such a bioelectrode 1 may be referred to as "bioelectrode assembly." FIG. 4 is a schematic perspective view of the bioelectrode 1 of FIG. 3 as viewed from the back side. In FIG. 3, under a state in which the protruding portions 32a of the connection terminals 32 of the electrode parts 30 are inserted through the through holes 22 of the substrate plate 20 and the protruding portions 32a are inserted through the through holes 12 of the fixing plates 10 so that the fixing plates 10, the substrate plate 20, and the three electrode parts 30 are assembled, each of the electrode parts 30 and its protruding portion 32a are movable within a range of the corresponding through hole 22 of the substrate plate 20. The protruding portion 32a of the connection terminal 32 of each electrode 30 can move laterally within a range restricted by a wall defining the through hole 22 of the substrate plate 20, and hence a distance between the connection terminals 32 of adjacent electrode parts 30 can be changed. Moreover, under the state in which the fixing plates 10, the substrate plate 20, and the three electrode parts 30 are assembled, each electrode part 30 can be rotated about its connection terminal 32 serving as an axis, and hence the positional relationship between the adjacent electrode parts 30 can also be changed. Under the state in which the fixing plates 10, the substrate plate 20, and the three electrode parts 30 are assembled, when, in plan view, as large a percentage as possible of the entire area of the conductive gel layer 35 of each electrode part 30 is arranged at a position not overlapping the substrate plate 20, the connection terminal 32 and the conductive gel layer 35 to be applied to a skin of a subject or a patient are separated from each other (have an offset structure), and hence noise is less liable to occur due to the movement of the living body or unevenness of the living body surface. Thus, this arrangement is preferred in view of achieving stable electrical connection of the connection part 32. It is more preferred that, in plan view, 50% or more of the entire area of each conductive gel layer 35 be arrangeable at a position not overlapping the substrate plate 20.

Next, the configuration of the electrode part 30 is described in detail. FIG. 5(A) is a plan view of one electrode part, and, in this embodiment, the electrode part 30 has, in plan view, a shape obtained by coupling a substantially rectangular part whose corners are rounded and a substantially circular part to each other. The connection terminal 32 is provided in the vicinity of a transition region of transitioning from the substantially rectangular part whose corners are rounded to the substantially circular part in a longitudinal direction of the electrode part 30, and is provided along a substantial center line in a width direction of the electrode part 30.

As illustrated in FIG. 5(B) and FIG. 6, each electrode part 30 includes the covering layer 31, the connection terminal 32, a sheet-shaped conductive layer 36, the conductive gel layer 35, an insulating layer 37, and a release film 38. The connection terminal 32 is conductive and passes through the covering layer 31. The conductive layer 36 serves as a second conductive layer, and is laminated in contact with the covering layer 31. The conductive gel layer 35 is in contact with the conductive layer 36 on a surface of the conductive layer 36 on a side opposite to the covering layer 31. The insulating layer 37 is laminated in contact with the conductive layer 36 on the surface of the conductive layer 36 on the side opposite to the covering layer 31, and covers a bottom plate portion 34b of a conductor 34 fitting to the connection terminal 32. The release film 38 covers the surface of the conductive gel layer 35. In this embodiment, the conductor 34 is a post of the hook.

A material of the covering layer 31 is not particularly limited as long as the material has an insulating property, and examples thereof include, but not limited to, a synthetic resin, glass, and paper. The covering layer 31 is preferably formed of a synthetic resin, and examples of the synthetic resin include: various resins including polyolefin resins, polyurethane resins, and polyester resins, such as polyethylene terephthalate (PET), polyethylene (PE), and polypropylene (PP); a foam body; non-woven fabric; woven fabric; and laminated products thereof. The covering layer 31 may be transparent or opaque, but the covering layer 31 is preferred to be opaque in view of design characteristics and covering components below the covering layer 31. The covering layer 31 can be colored as required to white, yellow, green, blue, red, brown, purple, gray, black, and other colors. The thickness of the covering layer 31 is not particularly limited, and is preferred to be, although the thickness depends on the material, for example, from 20 um to 200 um in a case of a resin film and from about 0.2 mm to about 1 mm in a case of a foam body.

In this embodiment, the connection terminal 32 formed of a stud forms a pair with the conductor formed of a post, and the connection terminal 32 includes a flat plate portion 32b and the protruding portion 32a extending from the flat plate portion 32b. The conductor 34 also includes a flat plate portion 34b and a protruding portion 34a extending from the flat plate portion 34b. The protruding portion 34a of the conductor 34 passes through the covering layer 31 and the conductive layer 36 and is fitted to a recessed portion formed inside of the protruding portion 32a. A lower surface of the flat plate portion 32b of the connection terminal 32 is brought into contact with the covering layer 31, and an upper surface of the flat plate portion 34b of the conductor 34 is brought into contact with the conductive layer 36. Thus, the covering layer 31 and the conductive layer 36 are firmly fixed to each other with the opposing surfaces of the connection terminal 32 and the conductor 34. The connection terminal 32 and the conductor 34 may each be made of a metal, or may each be made of a resin subjected to conductive coating. In order to suppress polarization, it is preferred to use a resin coated with silver-silver chloride or a carbon molded product coated with silver-silver chloride.

The connection terminal 32 and the conductive gel layer 35 are separated from each other, but the conductive layer 36 extends between the covering layer 31 and the conductive gel layer 35 under a state in which the conductive layer 36 is in contact with the connection terminal 32 and the conductive gel layer 35, and thus plays a role of electrically connecting the connection terminal 32 and the conductive gel layer 35 to each other. In this embodiment, the conductive layer 36 is laminated into a size substantially the same as that of the covering layer 31, but the size of the conductive layer 36 may be smaller than that of the covering layer 31, or this relationship may be reversed. When the electrode part 30 is viewed in plan view from the covering layer 31 side as in FIG. 5(A), the conductive layer 36 is invisible.

The conductive layer 36 can be formed of any material as long as the conductive layer 36 is an element (component) that is conductive. Examples of the material preferably include: a conductive film or sheet formed by mixing, in a resin, particles coated with conductive carbon, a metal, and/or a conductive polymer (for example, a resin coated with silver-silver chloride or a carbon molded product coated with silver-silver chloride); a material obtained by printing conductive ink on a resin film or sheet; and a metal sheet. When a base material of the conductive layer 36 is formed from a resin, the resin film or sheet is bent when the resin film or sheet is too soft, and does not fit along the skin when the resin film or sheet is too hard, leading to hindering of accurate measurement. Accordingly, it is preferred that the thickness of the conductive layer 36 be from 25 um to 180 um in total.

In terms of hardness and price, a material of the resin is preferably polyethylene (PE), polyurethane (PU), polyethylene terephthalate (PET), polypropylene (PP), or the like.

A film as used herein refers to a thin-film material having a thickness of less than 250 um, and a sheet as used herein refers to a thin-plate material of 250 um or more.

The insulating layer 37 is laminated in contact with the conductive layer 36 on the surface of the conductive layer 36 opposite to the surface thereof in contact with the covering layer 31, and covers the bottom plate portion 34b of the conductor 34 of the connection terminal 32. Accordingly, when the electrode part 30 is viewed in plan view from the insulating layer 37 side as in FIG. 5(C), the bottom plate portion 34b of the conductor 34 is invisible. The conductive layer 36 and the conductive gel layer 35 are required to be in contact with each other, and hence the insulating layer 37 extends from one end of the electrode part 30 in the longitudinal direction to include a portion around the connection terminal 32, to reach halfway in the longitudinal direction of the electrode part 30. The material of the insulating layer 37 is not particularly limited as long as the material has an insulating property, and various resin films including a polyolefin resin such as polyethylene and polypropylene, a polyvinyl chloride resin, a polyester resin, and a polyurethane resin can be used. The insulating layer 37 may be transparent or opaque, but the insulating layer 37 is preferred to be opaque in view of design characteristics and covering components below the insulating layer 37. The thickness of the insulating layer 37 is not particularly limited, and is, for example, from 10 um to 50 µm.

The conductive gel layer 35 extends in contact with the conductive layer 36 from an end in the longitudinal direction of the electrode part 30 on a side opposite to an end closer to the connection terminal 32, toward the end closer to the connection terminal 32. In a portion in which the insulating layer 37 is laminated on the conductive layer 36, the conductive gel layer 35 is laminated on the insulating layer 37. The conductive gel layer 35 is covered with the covering layer 31 through intermediation of the conductive layer 36. The connection terminal 32 and the conductive gel layer 35 are electrically connected to each other via the conductive layer 36, and hence an electrical signal acquired from a living body (animal, particularly human) is input from the connection terminal 32 of the bioelectrode 30 to an external device via a connection terminal of a connector of the external device.

The material of the conductive gel layer 35 is not particularly limited. The conductive gel layer 35 can be formed of, for example, an acrylic resin having conductivity imparted thereto, a urethane resin having conductivity imparted thereto, and the like, and is preferably an adhesive hydrogel containing an electrolyte. The adhesive hydrogel includes a polymer matrix obtained by copolymerization-crosslinking a monofunctional monomer which is an acrylic monomer and a crosslinkable monomer, water, a polyhydric alcohol, and an electrolyte. Examples of the acrylic monomer include a (meth)acrylamide-based monomer, a (meth)acrylic acid ester, and (meth)acrylic acid. Specific examples of the (meth)acrylamide-based monomer include (meth)acrylamide, an N,N-dialkyl(meth)acrylamide, an N-alkyl(meth)acrylamide, an N-hydroxyalkyl(meth)acrylamide, an N-alkoxyalkyl(meth)acrylamide, an amino group-containing cationic acrylamide-based compound, a sulfonic acid group-containing anionic monofunctional monomer or salts thereof, and derivatives thereof. The crosslinkable monomer is preferably a monomer having two or more polymerizable double bonds in a molecule thereof. Specific examples of the crosslinkable monomer include: polyfunctional (meth)acrylamides or polyfunctional (meth)acrylic acid esters, such as methylene bis(meth)acrylamide, ethylene bis(meth)acrylamide, (poly)ethylene glycol di(meth)acrylate, (poly)propylene glycol di(meth)acrylate, glycerin di(meth)acrylate, and glycerin tri(meth)acrylate;
tetraallyloxyethane; and diallyl ammonium chloride. The adhesive hydrogel may further contain a polyhydric alcohol, such as glycerin or polyethylene glycol, as a wetting agent. The adhesive hydrogel may contain other additives as required. Examples of the other additives include a rust inhibitor, a fungicide, an antioxidant, an antifoaming agent, a stabilizer, a surfactant, and a colorant. As such an adhesive hydrogel, various adhesive hydrogels are publicly known, including a conductive gel of the applicant. The thickness of the conductive gel layer 35 is not particularly limited, but is, for example, from 0.3 mm to 1.2 mm.

Under a state before the bioelectrode 1 is attached to the living body and used, the release film 38 is attached onto the conductive gel layer 35. With the conductive gel layer 35 being covered with the release film 38, the conductive gel layer 35 can be protected, and also can be protected from dryness. The release sheet 38 is formed into the same size as that of the conductive gel layer 35 or into a size slightly larger than that of the conductive gel layer 35. The conductive gel layer 35 is exposed when the release sheet 38 is peeled off, and hence the exposed conductive gel layer 35 can be brought into contact with the skin surface of the living body.

Referring back to FIG. 3, the bioelectrode 1 obtained by assembling the fixing plates 10, the substrate plate 20, and the plurality of electrode parts 30 can be mounted to the external device by electrically connecting the connection terminal 32 of the bioelectrode 30 and the connection terminal of the connector of the external device to each other. The connection terminal 32 in this embodiment forms a male hook, and hence can be easily designed so as to fit to an external device including a connection terminal formed of a female hook.

The bioelectrode of this embodiment can be used as an electrode for medical use such as: an electrode for low-frequency therapy; an electrode for an electrocardiogram (ECG); an electrode for biopotential measurement used for brain wave, eye nystagmus, electromyography, or the like; an electrode for electrical stimulation used for TENS, low-frequency therapy, or the like; a dispersive pad for an electric scalpel; and an electrode for iontophoresis. Examples of the corresponding external device include electric therapy equipment (including low-frequency therapy equipment, pain therapy equipment, and a non-invasive headache treatment device), electrocardiogram measuring equipment, a biopotential measurement device, a radiofrequency surgery device, an iontophoresis device, EMS equipment used for slimming and strength training, and a wearable vital sensor.

Now, effects of the bioelectrode 1 according to the above-mentioned embodiment are described below.

(1) In the bioelectrode 1 according to the above-mentioned embodiment of this invention, the diameter D₁ of each of the plurality of through holes 22 of the substrate plate 20 is larger than the diameter D₂ of the protruding portion 32a of the connection terminal 32, and is smaller than the diameter D₃ of the fixing plate 10. Accordingly, when the protruding portion 32a of the connection terminal 32 is inserted through the through hole 22 of the substrate plate 20, and then the fixing plate 10 is mounted on the protruding portion 32a, the electrode part 30 can be mounted to the substrate plate 20 without coming off from the substrate plate 20. Thus, the protruding portion 32a of the electrode part 30 is movable within the range of the through hole 22 of the substrate plate 20, and thus the distance between the protruding portions 32a of the connection terminals 32 of the adjacent electrode parts 30 can be changed. With such a configuration, the following effects are produced.
- The distance between the connection terminals 32 of the adjacent electrode parts 30 can be changed, and hence, at the time of mounting the bioelectrode 1 to the external device, the interval between the connection terminals 32 of the electrode parts 30 can be adjusted so as to match the interval between the connection terminals of the connectors of the external device.
Thus, the peel-off of the electrode is suppressed, and the stability of the electrically connected portion is improved. As a result, the measurement stability is improved.
·Moreover, the position of the electrode part 30 can also be changed by rotating the electrode part 30 about the protruding portion 32a of the electrode part 30 serving as an axis, and hence the position of the electrode part 30 can be adjusted in accordance with the site or the shape of the skin surface of the living body to which the bioelectrode 1 is applied. With appropriate arrangement of the electrode part 30 to the skin surface of the living body, the measurement accuracy is improved.
·The electrode parts 30 are independent of each other, and hence the electrode parts 30 can easily fit along the movement of the living body and the unevenness of the attaching surface.
·The plurality of electrode parts 30 form a set, and hence mounting and removing with respect to the external device are easier as compared to a case in which the electrode parts 30 are mounted to and removed from the external device one by one.
For example, when the electrode part 30 is removed from the connector of the external device, the electrode part 30 can be removed by pulling the substrate plate 20.
- The plurality of electrode parts 30 are removably mounted to the substrate plate 20 independently of each other, and hence the electrode part 30 can be individually replaced, and the entire bioelectrode 1 is not required to be replaced.
- The substrate plate 20 and the electrode part 30 mounted to the substrate plate 20 may be unremovable from each other by means of an adhesive or the like.
- No expanding/contracting part or a part to be cut is provided in the electrode part 30, and hence the electrode part 30 has less change and is stronger than the electrode part of the related art.
- Reduction in cost can be achieved by sharing the electrode part 30. As another example, the size and the shape of the electrode part 30 can also be easily changed.

(2) In the bioelectrode 1 according to the above-mentioned embodiment of this invention, the connection terminal 32 of each of the electrode parts 30 and the conductive gel layer 35 are separated from each other, and each of the electrode parts 30 further includes the conductive layer 36 extending between the covering layer 31 and the conductive gel layer 35 under a state in which the conductive layer 36 is in contact with the connection terminal 32 and the conductive gel layer 35.

With such a configuration, the connection terminal 32 and the conductive gel layer 35 applied to the skin of the subject or the patient are separated from each other (have an offset structure), and hence noise is less liable to occur due to the movement of the living body or unevenness of the living body surface, and thus the electrical connection of the connection part 32 becomes stable. Thus, the peel-off of the electrode is suppressed, and the stability of the electrically connected portion is improved. Thus, the measurement stability is improved.

(3) In the bioelectrode 1 according to the above-mentioned embodiment of this invention, each of the electrode parts 30 further includes the insulating layer 37 which is attached to the conductive layer 36, and which covers an end portion of the connection terminal 32 on a side opposite to the protruding portion 32a in a direction perpendicular to a direction in which the covering layer 31 extends.

With such a configuration, the electrical connection between the connection terminal 32 and the conductive gel layer 35 is more satisfactorily achieved. Further, the user of the bioelectrode 1 is prevented from coming into contact with the end portion of the connection terminal 32 on a side opposite to the protruding portion 32a.

(4) The bioelectrode 1 according to the above-mentioned embodiment of this invention includes the connection terminal 32 formed of a stud, and the conductor 34 formed of a post, the conductor 34 being fitted to the connection terminal 32.

With such a configuration, through use of publicly-known components, the connection terminal 32 can be easily manufactured.

(5) In the bioelectrode 1 according to the above-mentioned embodiment of this invention, when the protruding portion 32a of the connection terminal 32 is inserted through a corresponding one of the through holes 22 of the substrate plate 20, and the fixing plate 10 is fitted to the protruding portion 32a, under a state in which the electrode parts 30 are mounted to the substrate plate 20 so as to be unremovable from the substrate plate, in plan view, 50% or more of the area of the conductive gel layer 35 is arrangeable at a position not overlapping the substrate plate.

With such a configuration, the connection terminal 32 and the conductive gel layer 35 are offset from each other, and thus the electrical connection of the connection part 32 becomes stable. Further, the conductive gel layer 35 can be easily attached to the living body surface in accordance with the site or the shape of the skin surface of the living body to which the bioelectrode 1 is applied.

This invention has been described with reference to the embodiment illustrated in FIG. 2 to FIG. 6 as an example, but this invention is not limited thereto, and various modifications can be made thereto as follows.

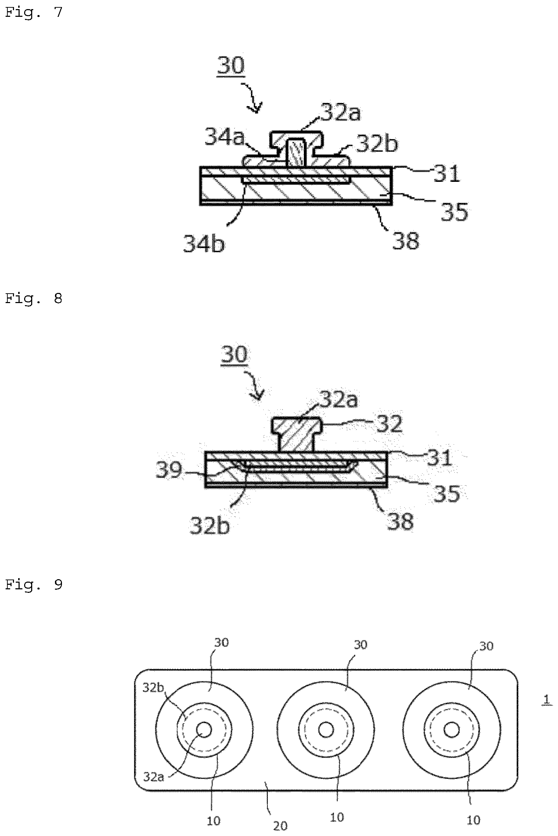

As illustrated in FIG. 7, the electrode part 30 is not required to include the conductive layer 36, and the connection terminal 32 and the conductive gel layer 35 may be arranged on the same axis, that is, at positions vertically overlapping each other through intermediation of the covering 31, in a direction perpendicular to a direction in which the covering layer 31 extends. In this embodiment, the end portion of the connection terminal 32 on the side opposite to the protruding portion 32a is buried in the conductive gel layer 35, and the electrical signal acquired from the living body is directly transmitted from the conductive gel layer 35 to the connection terminal 32. The insulating layer 37 may be omitted.

As illustrated in FIG. 8, the electrode part 30 is not required to include the conductor 34. In the case of this embodiment, the protruding portion 32a of the connection terminal 32 passes through the covering layer 31, and the flat plate portion 32b is in contact with the covering layer 31 in a part of the surface of the covering layer 31 in contact with the conductive gel layer 35. The flat plate portion 32b is fixed to the covering layer 31 by a pressure-sensitive adhesive sheet 39 which is laminated on this flat plate portion 32 and has a peripheral edge portion in contact with the covering layer 31, but the pressure-sensitive adhesive sheet 39 may be a pressure-sensitive adhesive, or the pressure-sensitive adhesive sheet 39 may be omitted. Further, in the embodiment illustrated in FIGS. 5, the electrode part 30 is not required to include the conductor 34.

The connection terminal 32 has been described as a stud in the embodiments of FIG. 2 to FIG. 8, but the connection terminal 32 is not limited thereto as long as a connection terminal that can be electrically connected to the connection terminal of the connector of the external device is used. Further, the conductor 34 has been described as a post, but the conductor 34 is not limited thereto as long as a member that can be engaged with the connection terminal 32 is used.

FIG. 9 is a schematic plan view of a bioelectrode in which three electrode parts 30 of FIG. 7 are integrated with the fixing plates 10 and the substrate plate 20 in a procedure similar to that of the embodiment illustrated in FIG. 2 to FIG. 6. In this embodiment, the three electrode parts 30 are arranged side by side in series, but the distance between the connection terminals 32 of the adjacent electrode parts 30 can be changed. Moreover, the position of the electrode part 30 can also be changed by rotating the electrode part 30 about the protruding portion 32a of the electrode part 30 serving as an axis. The electrode parts 30 are independent of each other.

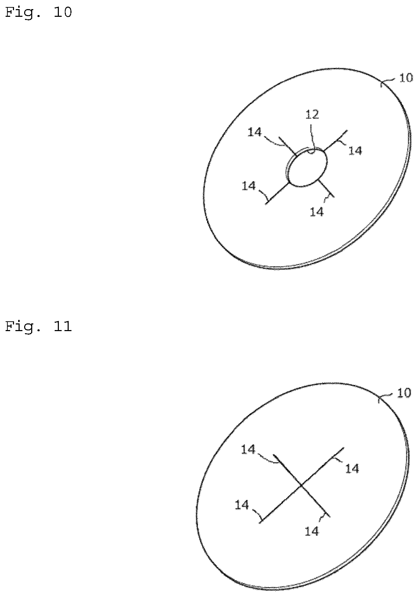

FIG. 10 is a schematic perspective view of the fixing plate 10 in another embodiment. In this embodiment, the fixing plate 10 has the through hole 12, and further has four slits 14 formed at equal intervals from the through hole 12 toward a radially outer side of the fixing plate 10. With such a configuration, even when the fixing plate 10 is harder and is thus less deformable or is thicker than the fixing plate 10 illustrated in FIG. 3, the through hole 12 slightly expands due to the slits 14, and the shape of the fixing plate 10 is restored as much as possible after the protruding portion 32a passes through the through hole 12 of the fixing plate 10. Thus, unless an external force is applied, the fixing plate 10 stops on the proximal end side with respect to the distal end of the protruding portion 32a, and is prevented from slipping out from the protruding portion 32a.

FIG. 11 is a schematic perspective view of the fixing plate 10 in another embodiment. The fixing plate 10 may have, in place of the through hole 12, another shape of a passing-through portion through which the protruding portion 32a of the electrode part 30 is inserted. For example, as illustrated in FIG. 11, the fixing plate 10 may have a cross-shaped or radial cut 14. Further, the passing-through portion is not limited to the cut 14, and the passing-through portion is only required to be formed at the middle of the fixing plate 10 so that this passing-through portion is plastically expanded to allow the protruding portion 32a of the electrode part 30 to be inserted therethrough.

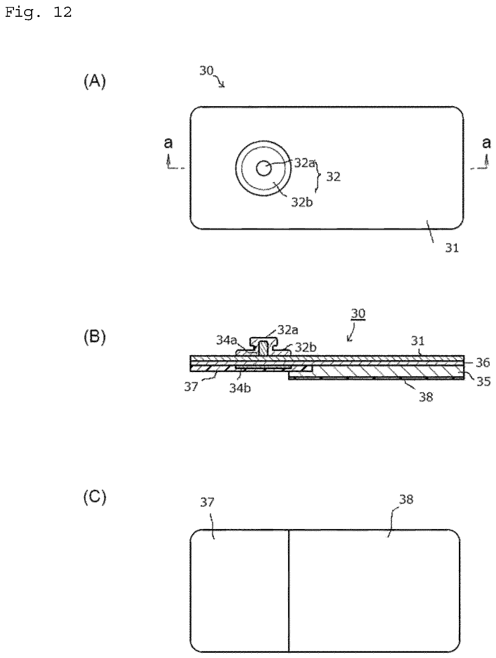

FIG. 12(A) to FIG. 12(C) are schematic perspective views of the electrode part 30 in another embodiment. In this embodiment, the electrode part 30 has, in plan view, a substantially rectangular shape whose four corners are rounded. The arrangement of the components of the electrode part 30 is the same as that described in the embodiment illustrated in FIGS. 5 and FIG. 6. Such an electrode part 30 having a substantially rectangular shape in plan view may be used in place of one, two, of three of the three electrodes 30 illustrated in FIG. 2.

In the embodiment of FIG. 2 to FIG. 6 and the embodiments of FIG. 7 and FIG. 8, in place of the conductive gel layer 35, a freely-selected conductive layer may be used as long as the conductive layer is to be arranged in contact with the living body (animal, particularly human) to transmit an electrical signal from/to the living body.

In place of the fixing plate 10 in the embodiment of FIG. 2 to FIG. 6 or the embodiment of FIG. 10, a perforated speed nut which is an integrally molded product of a metal may be used as the fixing plate. Further, a speed nut may be formed from a material other than a metal, such as a resin.

In the embodiment of FIG. 2 to FIG. 4 and the embodiment of FIG. 7, the number of electrode parts 30 in the bioelectrode 1 is three, but the number of electrode parts 30 is only required to be two or more, and may be two or four or more. Further, the arrangement of the connection terminals 32 of the plurality of electrode parts 30 is not limited to vertices of a triangle as illustrated in FIG. 3 or a side-to-side arrangement on a straight line as illustrated in FIG. 12(A) to FIG. 12(C). The number of fixing plates 10 and the number of through holes 22 formed in the substrate plate 20 are also not limited to three, and may each be two or four or more.

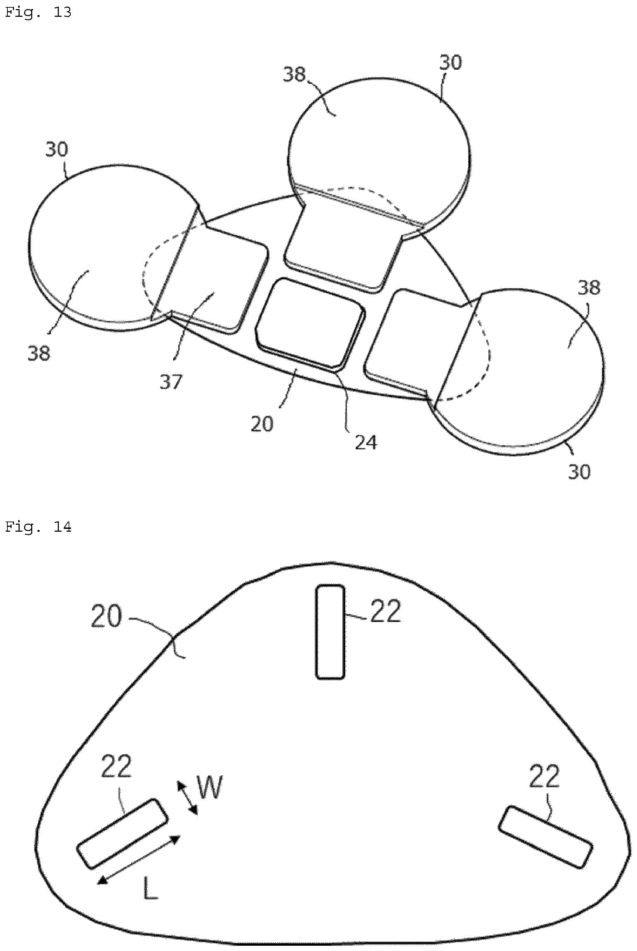

In any of the above-mentioned embodiments, as illustrated in FIG. 13, on a surface of the substrate plate 20 on the skin side, that is, on one of the wide surfaces of the substrate plate 20 on a side on which the electrode parts 30 are positioned, a non-slip material or a pressure-bonding adhesive layer 24 may be provided. The pressure-bonding adhesive layer 24 may be a publicly-known pressure-sensitive adhesive sheet or pressure-sensitive adhesive, or may be a pressure-sensitive gel. In FIG. 13, the pressure-bonding adhesive layer 24 is formed into a substantially quadrilateral shape on a part of the surface of the substrate plate 20 surrounded by the three electrode part 30, but this invention is not limited thereto. Fixing the electrode part 30 to a body of a wearer suppresses movement of the electrode part 30 due to body movement, and thus the electrode part 30 is less liable to be peeled off, but, with such a configuration, the substrate plate 20 can be fixed to the skin by the pressure-bonding adhesive layer 24. Further, the pressure-bonding adhesive layer 24 can temporarily fix the position of each electrode part 30 so as to prevent each electrode part 30 from moving. The substrate plate 20 and the electrode part 30 include the adhesive layer 24 having the pressure-sensitive adhesive property and the conductive gel layer 35, respectively, and hence the bioelectrode 1 is more firmly fixed to the skin surface of the body of the wearer, and the peel-off of the electrode part 30 from the skin surface is suppressed.

In any of the above-mentioned embodiments, as illustrated in FIG. 14, the through hole 22 of the substrate plate 20 may be shaped as a long hole in which a length L is longer than a width W. The hole may have a straight-line shape, an arc shape, a meandering shape, or a zigzag shape. A part or the whole of the width W of the through hole 22 along the length L of the substrate plate 20 is larger than the diameter D₂ of the protruding portion 32a of the connection terminal 32, and is smaller than the diameter D₃ of the fixing plate 10. With such a configuration, the movement of the protruding portion 32a is restricted by a wall defining the through hole 22, and hence the direction of the movement of the electrode part 30 can be restricted.

The connection terminal 32 and the connection terminal of the connector of the external device may be fitted to each other in any form. For example, a spring hook or a magnet hook may be employed.

This invention also encompasses a set for assembling a bioelectrode, which separately includes the fixing plate 10, the substrate plate 20, and the plurality of electrode parts 30 in any of the above-mentioned embodiments.

### Reference Signs List

1 ··· bioelectrode, 10 ··· fixing plate, 12 ··· through hole, 20 ··· substrate plate, 22 ··· through hole, 30 ··· electrode part, 31 ··· covering layer, 32 ··· connection terminal, 32a ··· protruding portion, 34 ··· conductor, 35 ··· conductive layer, 36 ··· conductive layer, 37 ··· insulating layer

## Claims

1. A bioelectrode, comprising:
a substrate plate having a plurality of through holes spaced apart from each other;
a plurality of electrode parts, each of the plurality of electrode parts including:
a covering layer;
a connection terminal which is conductive and passes through the covering layer, the connection terminal including a protruding portion protruding from the covering layer, the protruding portion passing through one of the plurality of through holes of the substrate plate; and
a conductive layer which is electrically connected to the connection terminal, and is to be brought into contact with a living body; and
a fixing plate mounted to the protruding portion of the connection terminal,
wherein a size of each of the plurality of through holes of the substrate plate is a size that allows the protruding portion of the connection terminal to be inserted therethrough, and is smaller than a size of the fixing plate.

2. The bioelectrode according to claim 1, wherein the each of the plurality of electrode parts is movable within a range of a corresponding one of the plurality of through holes of the substrate plate so that a distance between connection terminals of adjacent electrode parts is changeable.

3. The bioelectrode according to claim 1, wherein the connection terminal and the conductive layer of the each of the plurality of electrode parts are separated from each other, and the each of the plurality of electrode parts further includes a second conductive layer extending between the covering layer and the conductive layer under a state in which the second conductive layer is in contact with the connection terminal and the conductive layer.

4. The bioelectrode according to claim 3, wherein the each of the plurality of electrode parts further includes an insulating layer which is attached to the second conductive layer, and which covers a conductor fitted to the connection terminal or an end portion of the connection terminal on a side opposite to the protruding portion in a direction perpendicular to a direction in which the covering layer extends.

5. The bioelectrode according to any one of claims 1 to 4, wherein the connection terminal includes a male hook.

6. The bioelectrode according to any one of claims 1 to 4, wherein, when the protruding portion of the connection terminal is inserted through a corresponding one of the plurality of through holes of the substrate plate, and the fixing plate is fitted to the protruding portion, under a state in which the plurality of electrode parts are mounted to the substrate plate so as to be unremovable from the substrate plate, in plan view, 50% or more of an area of the conductive layer is arrangeable at a position not overlapping the substrate plate.

7. The bioelectrode according to any one of claims 1 to 4, wherein the conductive layer includes a conductive gel layer.

8. A set for assembling a bioelectrode, comprising:
a substrate plate having a plurality of through holes spaced apart from each other;
a plurality of electrode parts mountable to and removable from the substrate plate, each of the plurality of electrode parts including:
a covering layer;
a connection terminal which is conductive and passes through the covering layer, the connection terminal including a protruding portion protruding from the covering layer; and
a conductive layer which is electrically connected to the connection terminal, and is to be brought into contact with a living body; and
a fixing plate mountable to the protruding portion of the connection terminal,
wherein a size of each of the plurality of through holes of the substrate plate is a size that allows the protruding portion of the connection terminal to be inserted therethrough, and is smaller than a size of the fixing plate.
